# EUROPEAN PATENT APPLICATION

(11) **EP 0 586 355 A2**
(43) Date of publication of application: **09.03.1994**
(21) Application number: 93870173.7
(22) Date of filing: 18.08.1993
(51) Int. Cl.: C12N 15/82

(54) **Method for transforming monocotyledonous plants**

(30) Priority: 19.08.1992 US 932125
(71) Applicant: MONSANTO COMPANY, St. Louis Missouri 63167 (US)
(72) Inventor: Armstrong, Charles Lester, St. Charles, Missouri 63304 (US); Songstag, David Dean, Urbandale, Iowa 50322 (US)
(74) Representative: Nash, Brian Walter

(57) **Abstract**

A method for delivering foreign genetic material into immature embryos isolated from monocotyledonous plants, paticularly those of the Gramineae family is described. The method involves the isolation of immature embryos from the desired plant species, culturing the immature embryos for a period of time on a medium capable of inducing callus formation, introducing the foreign genetic material into the immature embryos and selecting transformed callus tissue to be regenerated into whole plants. Immature embryos that have undergone the culturing process prior to transformation exhibit a higher frequency of transformation when compared to that from freshly isolated immature embryos. The genetic material is preferably delivered by either electroporation or microprojectile bombardment methods.

## Description

### Field of the Invention

The present invention relates in general to plant genetic engineering and more particularly to an improved method for transforming immature embryos from monocotyledonous plants.

### Background of the Invention

The production of transgenic monocotyledonous plants is one of the primary research goals in plant biotechnology. Monocotyledonous plants, often referred to as "monocots," contain members of the Gramineae family which include some of the most agriculturally important cereal crops, such as corn, wheat, barley, rice, rye and sorghum (i.e. *Zea mays L., Oryza sativa L., Triticum aestivum L*). The introduction of transgenic cereal crops to the agricultural industry has been slow because of the difficulty in transforming these plants. Monocots, in general, have proved to be recalcitrant to transformation by *Agrobacterium* mediated DNA delivery; a method that has proved to be quite useful for the transformation of dicotyledonous plants. As a result, transformation methods not involving *Agrobacterium* have been pursued in an attempt to obtain transformed monocotyledonous plants. Some of these methods such as microprojectile bombardment, also known as particle-gun or biolistic technology (Fromm et al. 1990; Gordon-Kamm et al. 1990; Klein et al. 1988), electroporation (Dekeyser et al. 1990), DNA uptake during imbibition (Topfer et al. 1989) and violent mixture of plant cells with DNA and silicon carbide crystals (Kaeppler et al. 1990) have resulted in the successful transformation of some species of monocots.

Notwithstanding reports of the successful transformation of plant tissue from some monocot species by these various methods, the production of transgenic monocot plants, and their introduction to the farming industry, has continued to be quite problematic (Potrykus et al. 1990). This is partially the result of the difficulty in identifying a monocot explant that is suitable for both transformation and subsequent regeneration into fertile plants.

Success in producing transgenic maize has recently been reported utilizing embryogenic suspension cultures as the explant material in combination with the microprojectile bombardment method of transformation (Fromm et al. 1990; Gordon-Kamm et al. 1990). Even though transgenic maize plants were obtained by this method, the use of embryogenic cell suspension cultures as the explant is not always desirable because of the lengthy amount of time needed to establish a culture suitable for transformation. Moreover, it is well documented that the age of a cell culture is directly related to the occurrence of somaclonal variation and possibly culture-induced mutations. It would, therefore, be desirable to provide a method for transforming monocots that utilizes an explant that does not require several months for extensive cell culture development prior to transformation and that also reduces the risk of somaclonal variation or culture-induced mutations.

In view of the difficulty experienced in the science of obtaining these transgenic plants, any process that enhances the frequency of transformation would be beneficial to the ultimate goal of efficient production of transgenic monocotyledonous plants. Therefore, a need exists in the field of plant biotechnology for an explant from monocotyledonous plants that is amenable to transformation and subsequent regeneration into a whole plant and a transformation method that enhances the frequency of transformed tissue that can be regenerated into transformed plants.

### Summary of the Invention

The present invention involves a method for delivering foreign genetic material into immature embryos isolated from monocotyledonous plants, particularly those of the Gramineae family. The method comprises isolating immature embryos from the desired plant species, culturing the immature embryos from 1 to 14 days on a medium capable of inducing callus formation, introducing the foreign genetic material into the immature embryos, and selecting transformed callus tissue to be regenerated into whole plants. The foreign genetic material is preferably delivered by either the electroporation or microprojectile bombardment methods. Quite unexpectedly, immature embryos that have undergone the culturing process prior to transformation exhibit a higher frequency of transformation when compared to that from freshly isolated immature embryos.

It is therefore an object of the present invention to provide a method for delivering foreign genetic material into monocotyledonous plants that enhances the frequency of transformation.

It is further an object of the present invention to provide an explant from species of plants of the Gramineae family that can be transformed and subsequently regenerated into whole plants.

Other and further objects and advantages of the invention will become evident to those skilled in the art from the following specification.

### Brief Description of the Drawings

Figure 1 is a physical map of pMON19433.

Figure 2 is a physical map of pBC17.

Figure 3 is a physical map of pEC9.

Figure 4 is a physical map of pMON8678.

### Detailed Description of the Preferred Embodiments

It has been discovered that genetic material may be delivered into an explant comprising immature embryos isolated from species of monocotyledonous plants and that whole plants can be regenerated therefrom. The frequency of transformation is enhanced by culturing the immature embryos from between 1 to 14 days on a medium capable of inducing callus formation. In accordance with the present invention, DNA containing a desired structural gene is delivered into immature embryos that have undergone a culturing treatment prior to transformation and stably transformed plants are obtained.

The immature embryo explant that serves as the starting material for the production of a transgenic monocotyledonous plant by the method of this invention is isolated from the developing plant of the desired species. As used herein, the immature embryo explant is an intact, meristematic tissue that has differentiated to approximately a "Stage 1" embryo according to the classification scheme of Abbe and Stein (1954). The immature embryo explant is capable of cell division to give rise to callus cells that can differentiate to produce the definitive tissues and organs of a whole plant.

Immature embryos from a monocotyledonous plant are isolated from the fertilized reproductive organ of the desired plant species. Methods for isolating immature embryos from the various monocotyledonous plants are known and described in the literature, e.g. wheat (Maddock et al. 1983), rye (Lu et al. 1984), barley (Thomas and Scott 1985), rice (Peng and Hodges 1989) and corn (Green and Phillips 1975). For exemplary purposes, maize immature embryos may be isolated from pollinated plants. The plants are pollinated by any method known in the art such as that described by Neuffer et al. (1982). Approximately ten days after pollination, immature embryos ranging from about 1.0 to 2.0 mm in length are contained in the developing ear. These immature embryos are aseptically isolated from the developing ear and used as the explant for the method of the present invention.

In accordance with the method of the present invention, the frequency with which the immature embryos are transformed is enhanced by culturing the immature embryos prior to transformation. Culture media capable of inducing embryogenic callus formation are suitable for the culturing step. The immature embryos are preferably cultured on a medium such as N6 1-100-25 with or without the addition of silver nitrate. N6 1-100-25 medium contains the N6 salts and vitamins (Chu et al. 1975), 1.0 mg/L 2,4-D, 100 mg/L casamino acids, 20g/L sucrose and 25 mM proline. When AgNO₃ is used in the medium, it is added at a concentration from about 10 µM to about 100 µM. This medium is referred to as N6 1-100-25-Ag medium. Other suitable culture media are known to those skilled in the art and can be advantageously used in the culturing step of this invention.

The immature embryos may be cultured from 1 to about 14 days in the dark at 28°C on the desired culture medium. It has been found that culturing the immature embryos for at least one day and not more than fourteen days provides an enhanced frequency of transformation resulting in a significant number of transformation events. Preferably, the immature embryos are cultured from one to eight days. Most preferably, the immature embryos are cultured from 1 to 6 days. A four day culture period on N6 1-100-25 or N6 1-100-25-Ag medium has been particularly effective. In general, the immature embryos may be cultured prior to transformation for a period of time until embryogenic callus formation is visibly evident. Although not intending to be bound by this mechanism, it is believed that the cell walls of the immature embryos become altered in some manner during the culturing period which permits enhanced delivery of DNA into these tissues when electroporation is used as the means for delivering the DNA to the cells.

The desired genetic material may be delivered to the cultured immature embryos of the present invention by any transformation method, but the microprojectile bombardment or electroporation methods are preferred. Protocols for the introduction of DNA into plants by either of these two methods are known to those skilled in the art. It is understood that these protocols may be adapted or modified for use with particular monocot species and that such adaptations or modifications are within the scope of the present invention.

Generally, the microprojectile bombardment method involves coating the desired heterologous DNA onto a suitable microprojectile, such as tungsten or gold, and delivering the microprojectiles to the plant tissue. Any of several known and routine methods such as those described in Klein et al. 1989; Oard et al. 1990; Fromm et al. 1990; and McCabe et al. 1988 may be used. The microprojectiles are introduced into the cultured immature embryos by a microprojectile gun device. The design of the accelerating device or gun is not critical so long as it can perform the acceleration function without excessive damage to the target tissue. The accelerated microprojectiles impact upon the cultured immature embryos to perform the delivery of the desired DNA.

Although any microprojectile gun device may be used, a modified Biolistic® PDS 1000 microprojectile device has been used effectively for delivering DNA into immature embryos according to the method of this invention. This device has a stopping plate configuration similar to commercially available stopping plates except that the lexan disk is 3/8" thick with a 3/32" diameter hole through the disk center. The hole is enlarged at the upper surface to 7/16" and this tapers in a countersunk arrangement to a depth of 1/4" at which point it narrows to the 3/32" diameter hole which does not have a taper for the remaining 1/8" thickness. The petri plates containing the cultured immature embryos are placed at level 4 of this device which is one level from the bottom. The immature embryos are typically subjected to one shot from the devise. A shielding metal screen with 100 µ openings is typically used on the shelf position immediately below the stopping plate. The process is performed under a suitable vacuum. While this microprojectile device and its operating parameters have been described with particularity, it should be understood that other microprojectile devices having different operating parameters would be useful in delivering DNA to the cultured immature embryos of this invention.

Another useful method for delivering DNA into the cultured immature embryos of the present invention is by electroporation. In general, electroporation is a method of direct DNA transfer whereby the desired DNA is delivered into the tissue to be transformed by causing an electrical discharge in the presence of the tissue and the DNA. While known methods for electroporation may be used in conjunction with the method of the present invention, optimal electroporation parameters for use in electroporating cultured immature embryos have been identified and are discussed below.

For optimal transformation of cultured immature embryos by electroporation, the immature embryos are removed from the culture medium and floated on electroporation buffer [(EPR) which contains a solution of 10% glucose, 4 mM CaCl₂, 10 mM Hepes, pH 7.2, DeKeyser et al. (1990)] containing 0.2 mM spermidine for 3 hours. This solution is removed and replaced with fresh EPR buffer at hourly intervals. The immature embryos are then placed into an electroporation cuvette containing 150 µl EPR buffer with 70 mM sodium glutamate. The number of immature embryos per cuvette may range between 1 and 20, but superior results have been obtained with an immature embryo density of 5 per cuvette. The electroporation is performed at room temperature (∼22°C) after a 10 minute heat shock at 37°C. A single electric pulse is delivered with a field strength of 375V/cm (field strength = voltage applied/width of electrode gap) and a capacitance setting of 960 µF.

In the Examples that follow which employ the electroporation method of DNA delivery, the electroporation was conducted using a Gene Pulser® Transfection Apparatus from Bio-Rad (Bio-Rad Laboratories, Richmond CA). The Gene Pulser® apparatus is a pulse generator which uses capacitor discharge to produce controlled exponential pulses. The capacitance setting, the initial voltage and the electrode gap are selected by the user. The unit automatically measures and displays the actual voltage delivered and the resistance-capacitance (RC) time constant. The Capacitor Extender package (Bio-Rad) was used. Gene Pulser® cuvettes of 0.2 cm or 0.4 cm electrode gap were used. The electrodes are contained in the cuvette.

As is understood by those in the art, other and different electroporation parameters may be used advantageously in conjunction with the method of the present invention. The EPR buffer may contain other electrolyte salts such as NaCl, KCl or potassium glutamate, which may be present in various concentrations. Successful electroporation has been achieved using concentrations ranging from about 0.035 M to about 0.15 M. Immature embryos from maize that are exposed to a lower salt concentration have a generally higher frequency of transformation. This may be due to the increased pulse length that occurs in the presence of lower ionic strength solutions. In addition, non-chloride organic acid salts are preferred. Increasing the field strength to 750 V/cm or higher is generally detrimental, but some transformation does occur at this level. A single electrical pulse was also found to work better than five consecutive pulses.

Once the immature embryo has been transformed, callus cultures are established. Typically, the embryos are washed several times with liquid N6 1-100-25 medium or other suitable liquid medium to remove any residual EPR buffer and then replated onto 0.2% Phytagel®-solidified N6 1-100-25 medium with or without silver nitrate or other suitable medium for inducing embryogenic callus formation. Embryogenic callus forms on this media. A desired selection agent may be added to this medium to select transgenic cells and to suppress growth of non-transgenic cells.

Whole plants are regenerated from the callus obtained from the transformed immature embryos by any suitable method. Plant regeneration from callus cultures can be accomplished in a variety of manners known to those skilled in the art. Regeneration protocols that are suitable for particular members of the Gramineae family are known and available. In general, regeneration can be accomplished by first incubating the callus tissue on a medium that promotes some type of embryo differentiation. The callus tissue is then placed on a medium that promotes embryo enlargement and maturation and, finally a medium that promotes germination. The first two steps are generally carried out in the dark at about 28°C, whereas the germination step is generally carried out under a 16:8 hour photoperiod at about 25°C. Shoots formed on the germination medium are transferred to tubes or trays still containing the germination medium to permit further plant development and root formation. Once a good root system has developed, the plants are carefully removed from the medium, the root system washed thoroughly under running water, and the plants placed into pots containing Metromix® 350 growing medium, or other suitable soil mix. The freshly transplanted plants are maintained in a high humidity environment for several days. After several days, the humidity should be gradually reduced to harden off the plants. Once hardened off, the plants can be transplanted to larger pots for growth chamber or greenhouse conditions.

While the above general protocol is particularly suited to the regeneration of corn, similar protocols are available for regenerating other species of monocot plants.

Any desired genetic material may be inserted into the cultured immature embryos of the desired plant species. The genetic material may be in the form of a plasmid or a DNA fragment The plasmid or DNA fragment will preferably contain a desired gene under the transcriptional control of regulatory sequences capable of causing sufficient expression of the desired gene in a monocot plant and other elements necessary for the expression of the desired gene in a plant. Typically, to obtain expression of a heterologous or foreign gene in a monocotyledonous plant, the genetic material inserted into the plant cells comprises a promoter, an intron from a gene from a species of monocots, a foreign structural gene, and a 3' polyadenylation signal. As used herein, a "foreign" or "heterologous" gene means a structural coding sequence not normally associated with the promoter used to drive expression of that coding sequence. The term "gene" means coding region together with 5' and 3' sequences. The term "structural gene" or "structural DNA sequence" means coding region.

The DNA fragment or plasmid also preferably contains one or more reporter genes so that transformed tissue may be observed, selected and/or scored. Examples of suitable reporter genes include, but are not limited to, the β-glucuronidase gene (GUS), genes providing anthocyanin expression, a gene providing luciferase expression, antibiotic resistance genes such as the neomycin phosphotransferase gene (NPTII), the hygromycin phosphotransferase gene (HPT), and the spectinomycin/streptomycin gene, or herbicide resistance genes such as a variant 5-enolpyruvylshikimate 3-phosphate synthase (EPSPS) for selection by resistance to the herbicide glyphosate, the phosphinothricin acetyltransferase gene which confers resistance to phosphinothricin (the active ingredient in the herbicide Basta®,), or a variant acetolactate synthase (ALS) gene which confers resistance to chlorsulfuron.

Plasmids or DNA fragments containing the desired genetic elements to be inserted into the cultured immature embryo can be prepared by standard methods known to those skilled in the art. Generally, DNA manipulations to prepare vectors for transformation into plants are performed following the procedures described in Sambrook et al. (1989).

The method of the present invention may be utilized to obtain transgenic plants of any species of monocot plants, particularly members of the Gramineae family such as corn, wheat, barley, rye, rice and sorghum. In particular, the methods of the present invention are useful in producing transgenic corn. Any corn genotype capable of efficient regeneration from immature embryos may be used. Immature embryos obtained from a high type-II genotype of corn, such as that derived from inbred lines A188 and B73 (Armstrong et al. 1991), are particularly preferred.

In use, immature embryos from a species of the Gramineae plant family are isolated and cultured from between 1 to 14 days, preferably from 1 to about 6 days, and transformed by either the microprojectile bombardment or electroporation method with desired genetic material. Immature embryos that have been transformed are selected and regenerated into whole plants. These regenerated transgenic plants confer the desired trait or characteristic from the inserted gene or genes to the plant.

The following examples are provided to illustrate the methods of the present invention and should not be interpreted in any way to limit the scope of the invention. Those skilled in the art will recognize that various modifications can be made to the methods described herein while not departing from the spirit and scope of the present invention.

### EXAMPLE 1

Immature embryos from a high type-II (Hi-II) *Zea mays L.* genotype (Armstrong et al. 1991) derived from inbred lines A188 and B73 were collected from self-pollinated primary ear shoots. These immature embryos were cultured for either 0, 1, 4, or 14 days on N6 1-100-25-Ag medium in the dark at 28°C. The cultured maize immature embryos were then electroporated in EPR buffer containing 150 mM NaCl and 20 µg of either plasmid pMON19433 or pBC17. The electroporation was performed in 0.8 ml BioRad cuvettes using the Gene Pulser® Transfection Apparatus from BioRad Laboratories with a field strength of 375 V/cm and a capacitance setting of 960 µF. The cuvettes containing the immature embryos in the EPR buffer with the desired plasmid were placed on ice for 10 minutes prior to electroporation.

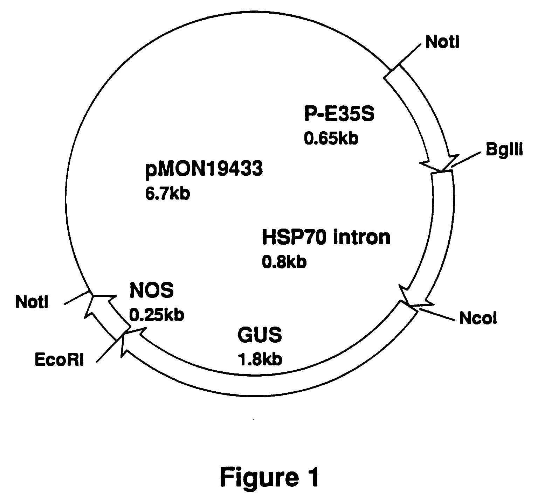

Plasmid pMON19433 was engineered specifically for transformation and expression in corn. A physical map of pMON19433 is presented in Figure 1. Functionally, pMON19433 contains a β-glucuronidase reporter gene comprised of the enhanced 35S promoter from cauliflower mosaic virus (P-e35S), the heat shock protein 70 intron from corn (HSP70 intron), the β-glucuronidase structural coding region (GUS: 1) and a 3' polyadenylation signal from the nopaline synthase gene of the Ti plasmid of *Agrobacterium tumefaciens* (NOS 3'). The vector pMON19433 also contains the gene conferring resistance to ampicillin (AMP) and origins of replication for the pUC plasmids and the M13 phage.

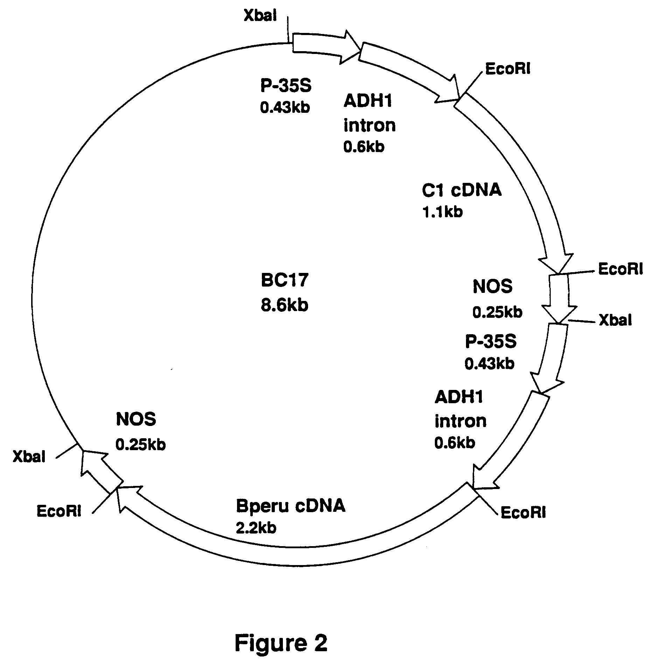

Plasmid pBC17 was also engineered specifically for expression in corn and expresses the cDNAs of two naturally occurring genes which regulate anthocyanin biosynthesis in corn cells. A physical map of pBC17 is presented in Figure 2. Both corn anthocyanin regulatory genes are driven by the 0.43 kb 35S promoter from the cauliflower mosaic virus (CaMV35S pro; Odell et al. 1985) which is joined to the 0.58 kb corn alcohol dehydrogenase 1 intron 1 fragment to provide increased levels of gene expression (ADH1 intron; Callis et al. 1987). The 3' end of the gene is the 0.3kb nopaline synthase gene 3' end (NOS 3') (Fraley et al. 1983). The CaMV 35S promoter and Adh1 intron are attached to the C1 cDNA which is derived from the corn C1 anthocyanin gene (Goff et al. 1991) for one of the reporter genes and to the cDNA of the B^{Peru} anthocyanin gene (Goff et al. 1990) for the other reporter gene. The remainder of the plasmid is the pUC plasmid as described by Vierra and Messing (1982).

Following electroporation, the immature embryos were assayed for expression of the GUS gene by assaying in the presence of X-gluc as described by Jefferson et al. (1987), or by analyzing the transformed tissue for red anthocyanin spots indicative of expression of the anthocyanin regulatory genes. The results of four replications of this experiment are presented in Table 1 below.

**TABLE 1**

| Days of Culture | GUS Spots per 20 embryos | Anthocyanin Spots per 20 embryos | Pulse Length (msec) |
|---|---|---|---|
| 0 | 0.6±0.3 | ND* | 80 |
| 1 | 2.0±0.9 | ND | 80 |
| 4 | 3.3±1.4 | 3.5±0.5 | 90 |
| 14 | 0 | ND | 85 |

| | | | |
|---|---|---|---|
| * ND = not determined | | | |

The values in Table 1 represent average GUS or anthocyanin spots per 20 embryos ± standard error. This example illustrates that culturing the immature embryos prior to transformation increases the frequency of transformation of immature embryos from corn.

### EXAMPLE 2

Transient expression of a reporter gene introduced into corn immature embryos by transformation either immediately after isolation (Day 0 embryos) or after a 4-day preculture treatment (Day 4 embryos) was determined and compared.

A sib-pollinated "Hi-II" ear (A188/B73 derivative; Armstrong et al. 1991) was harvested 8 days after pollination, refrigerated overnight at 4°C, surface sterilized for 20 minutes in half-strength Chlorox bleach plus a few drops of Tween 20, and rinsed in two changes of sterile deionized water. One hundred immature embryos, about 1 to 2 mm in length, were aseptically isolated for this experiment.

For the "Day 0" treatment, 50 embryos were placed directly into EPR buffer plus 0.2 mM spermidine in BioRad electroporation cuvettes. One hundred fifty µl of EPR buffer and 5 freshly isolated immature embryos were added per 0.8 ml cuvette. A total of ten cuvettes were prepared. Twenty µl of CsCl₂-purified plasmid DNA at a concentration of one µg/µl in TE (10 mM Tris, 1.0 mM EDTA) buffer was added to each cuvette. The DNA used was plasmid pMON19433 as described in Example 1. Following incubation at room temperature for one hour, 9 µl of a 1.7 M sodium glutamate stock was added to each cuvette. After an additional ten minutes at room temperature (22°C), the embryos were electroporated at 150V with a 960 µF capacitance setting. Following a final ten minute incubation at room temperature (22°C), the buffer was carefully pipetted out of the cuvettes, and the embryos rinsed by flooding the cuvettes with sterile liquid N6 1-100-25 medium. The embryos were then plated onto Phytagel®-solidified N6 1-100-25-Ag (10 µM silver nitrate) medium and incubated in the dark at 28°C. Approximately 48 hours later, the embryos were assayed histochemically for β-glucuronidase activity by immersing in X-gluc and incubating for 24 hours at 37°C. The number of blue spots per embryo were counted to measure the efficiency of DNA transfer and expression.

For the "Day 4" treatment, 50 embryos were placed onto Phytagel®-solidified N6 1-100-25-Ag (10 µM silver nitrate) medium and incubated in the dark at 28°C. Four days later, the embryos were removed from the culture medium and placed into EPR buffer in BioRad electroporation cuvettes. One hundred fifty µl of EPR buffer and 5 four-day cultured embryos were added per 0.8 ml cuvette for a total of ten cuvettes. The same protocols for DNA addition, electroporation and histochemical assays were followed as for the "Day 0" embryos. Plasmid pMON19433 was again used as the transformation vector.

The results of this experiment are summarized in Table 2. A four-fold improvement in transient GUS expression was observed by preculturing the embryos for four days on N6 1-100-25-Ag (10 µM silver nitrate) medium.

**TABLE 2**

| Rep # | # blue spots / embryo | |
|---|---|---|
| | Day 0 | Day 4 |
| 1 | 1.8 | 13.0 |
| 2 | 3.0 | 14.8 |
| 3 | 3.0 | 2.8 |
| 4 | 0.8 | 6.8 |
| 5 | 3.4 | 8.6 |
| 6 | 1.6 | 8.0 |
| 7 | 1.8 | 9.6 |
| 8 | 1.4 | 13.4 |
| 9 | 3.2 | 8.4 |
| 10 | 3.6 | 16.0 |
| Overall mean ± std error of mean: | 2.4±.03 | 10.1±1.3 |

### EXAMPLE 3

Immature embryos from corn, 1-2 mm in length, from the "Hi-II" genotype as described in Example 1 were isolated onto Phytagel®-solidified N6 1-100-25-Ag medium (50 µM silver nitrate). Embryos were transformed by the microprojectile bombardment method on either Day 0 (the day of embryo isolation), or 1, 2 or 4 days after embryo isolation and culture in the dark on N6 1-100-25-Ag medium. The embryos were bombarded with tungsten microparticles carrying a mixture of plasmids pEC9 and pMON8678.

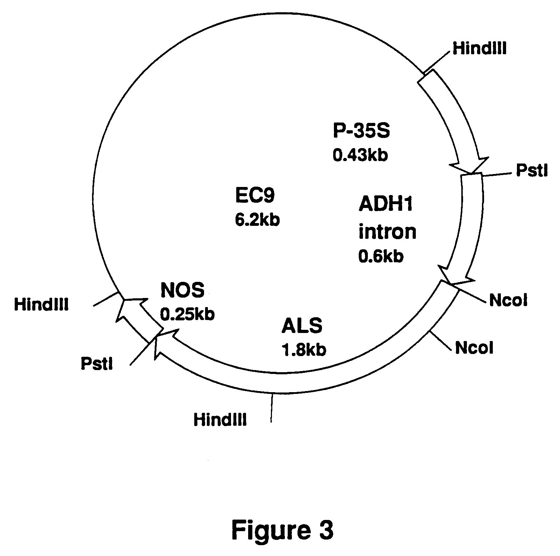

Plasmid pEC9 is a transformation vector that has been particularly constructed for use in transforming maize. A structural map of pEC9 is presented in Figure 3. Plasmid pEC9 includes, as a selectable marker gene, the 35S promoter from cauliflower mosaic virus (P-35S) attached to intron one from the maize alcohol dehydrogenase 1 gene (ZmADH1S*int1) driving expression of a chlorsulfuron tolerant form of the maize enzyme acetolactate synthase gene (pre ZmALS). The mutations necessary to the ALS gene to render it able to confer chlorsulfuron tolerance are described in Haughn (1987) and Lee (1988). The gene is terminated by the NOS 3' polyadenylation region from *Agrobacterium tumefaciens* (NOS 3').

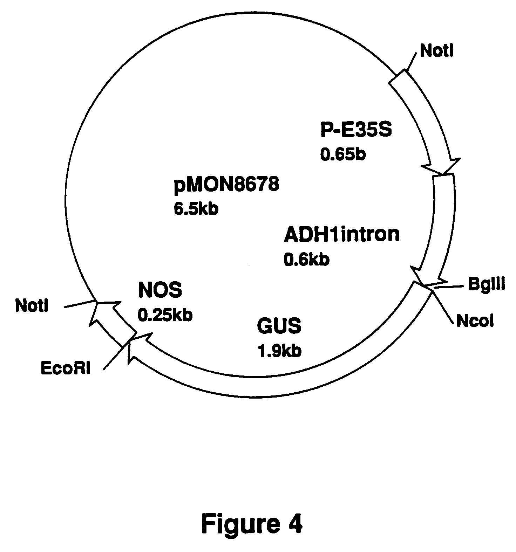

Plasmid pMON8678 has also been particularly constructed for expression in corn and its physical map is presented in Figure 4. Plasmid pMON8678 contains the enhanced 35S promoter from a cauliflower mosaic virus (P-e35S) attached to intron one from the maize alcohol dehydrogenase one gene (ZmADH1S*int1) driving expression of the gene encoding β-glucuronidase (GUS:1). This gene is terminated by the NOS 3' polyadenylation region (NOS 3').

Microprojectiles carrying a mixture of plasmids pEC9 and pMON8678 were introduced into the immature embryos using the modified Biolistic® PDS 1000 particle accelerator device, as previously described, and standard DNA precipitation conditions (Klein et al. 1989). Several plates of embryos were maintained as non-bombarded controls. After a total of two weeks in culture on N6 1-100-25-Ag medium, type-II culture response was scored. The results of the type-II culture response are presented below in Table 3.

**TABLE 3**

| | Control Day (not bombarded) | 0 Day 0 | Day 1 | Day 2 | Day 4 |
|---|---|---|---|---|---|
| Rep 1 | 19/20* | 10/30 | 7/30 | 19/30 | 19/20 |
| Rep 2 | 28/30 | 1/20 | 1/30 | 7/25 | 19/20 |
| Rep 3 | 18/20 | 0/20 | 3/30 | 21/30 | 27/30 |
| Rep 4 | | 1/20 | 7/30 | 22/30 | 22/30 |
| Rep 5 | | | 12/30 | 25/30 | 18/20 |
| Mean % ±SE | 93±1 | 11±8 | 20±6 | 64±9 | 92±1 |

| | | | | | |
|---|---|---|---|---|---|
| * Number of embryos forming type-II callus/total number of embryos. | | | | | |

The data in Table 3 shows that the culture response from corn immature embryos bombarded after a four day preculture period on N6 1-100-25-Ag (50 µM silver nitrate) medium was comparable to that of non-bombarded controls. Significantly reduced culture initiation frequencies were obtained with immature embryos bombarded on the same day as the embryos were isolated (Day 0). A significantly enhanced frequency of transformation was evident when transformed after 2 or 4 days of culture. Selection for transformed callus was started about two weeks after embryo isolation by transfer onto N6 1-0-25 medium (without casamino acids) containing 50 µM of silver nitrate and 20 ppb chlorsulfuron. After two weeks on this medium, the cultures were transferred again onto fresh N6 1-0-25-Ag-C20 medium containing 50 µM silver nitrate and 20 ppb chlorsulfuron. Five weeks later (a total of 64 days after embryo isolation), one rapidly growing chlorsulfuron-resistant callus sector was observed from a Day 4 embryo. A sample of this tissue was placed into X-gluc, and within four hours a large proportion of the tissue had turned blue, indicating stable transformation and expression of the GUS gene from plasmid pMON8678. The remaining tissue from this transgenic cell line was transferred to fresh N6 1-0-25-Ag-C20 medium containing 50 µM silver nitrate and 20 ppb chlorsulfuron to bulk up the tissue for further studies. Two weeks later, a portion of this callus was transferred to MS medium with 0.1 mg/l 2,4-D and 0.1 µM ABA to initiate plant regeneration. After an additional transfer to N6 medium containing 6% sucrose and no plant growth regulators, regenerated plants developed. One of these plants was incubated in X-gluc and within two hours the root tips turned blue. After overnight incubation at 37°C, nearly all of the plant tissue was dark blue, indicating that this plant was stably transformed with and expressing the GUS gene from pMON8678.

After a total of 79 days from embryo isolation, a second chlorsulfuron resistant sector of tissue was observed from a Day 2 immature embryo. A sample of this tissue was placed into X-gluc and it also turned dark blue within a few hours.

After a total of 120 days from embryo isolation, a third chlorsulfuron-resistant sector of tissue was observed from another embryo from a Day 2 immature embryo. This tissue also exhibited GUS expression by a positive staining with X-gluc.

### EXAMPLE 4

Corn immature embryos, 1 to 2 mm in length, from a "Hi-II" genotype as described in Example 1, were isolated onto Phytagel®-solidified N6 1-100-25-Ag medium containing 10 µM silver nitrate. The immature embryos were cultured on this medium for four days prior to bombardment. Approximately 960 embryos were bombarded. A 1:1 mixture, by weight, of plasmids pEC9 and pMON19433, as previously described, was precipitated onto either DuPont 1.0 µ gold or M10 tungsten particles and then delivered into the cells using a modified Biolistic® PDS 1000 particle gun as previously described. Selection was initiated two weeks after bombardment by transferring calli onto Phytagel®-solidified N6 1-0-25-Ag-C20 medium containing 20 ppb chlorsulfuron, with 10 µM silver nitrate. Selection was continued for about two months, with transfer of growing tissue onto fresh selection media every two weeks.

A total of sixteen independently confirmed transgenic cell lines were recovered from this experiment. All sixteen were chlorsulfuron-resistant and also positive in PCR assays for the presence of the introduced DNA sequences. The primers used for PCR assays were specific for portions of the 35S promoter and the Adhl intron 1, which are present in both pEC9 and pMON19433. Eight of the sixteen lines also expressed the GUS gene from pMON19433 as determined by histochemical staining of tissue samples in X-gluc.

Plants were regenerated from 5 of the 16 transgenic cell lines obtained. Plants from 2 of these 5 cell lines were verified to express the β-glucuronidase gene by X-gluc histochemical analysis of leaf tissue.

### EXAMPLE 5

Corn immature embryos, 1 to 2 mm in length, from the Hi-II genotype as described in Example 1 were placed onto Phytagel®-solidified N6 1-100-25-Ag medium containing 10 µM silver nitrate. Embryos were bombarded five days after isolation with a mixture of pEC9 and pMON19433. These plasmids were as previously described.

Each DNA mixture was precipitated onto M10 tungsten particles and shot into the embryos using the modified Biolistic® PDS 1000 particle gun having the operating parameters as described above. The embryos were transferred immediately after bombardment onto fresh N6 1-100-25-Ag medium containing 10 µM silver nitrate. Selection was initiated two weeks later by transferring tissue onto N6 1-0-25-Ag-C20 medium with 20 ppb chlorsulfuron and 10 µM silver nitrate. Selection was continued by transferring growing tissue to fresh selection medium every two weeks. Fifty-two days after bombardment, two rapidly growing chlorsulfuron-resistant sectors of tissue were identified, tracing back to two different embryos. Samples from each were stained with X-gluc and one turned dark blue within hours (GUS positive - cell line T21-1) and the other exhibited only scattered areas of blue tissue (cell line T17-1). PCR analysis of T17-1 indicated that it was indeed transformed. Primers used for the PCR analysis were for the CaMV35S promoter and the ADH1 intron 1, which are present in both pEC9 and pMON19433. Plants were regenerated, under 20 ppb chlorsulfuron selection pressure, from both of these cell lines.

GUS expression (based on histochemical staining with X-gluc) was observed in a wide range of tissues in the regenerated plants from both cell lines, including leaf blade and sheath, root, stalk, silk, and pollen. No GUS expression was observed in comparable non-transgenic control corn tissues incubated in X-gluc at the same time as the tissues from plants from cell lines T21-1 and T17-1. Plants from these lines were completely fertile, and both self-pollinations and outcrosses to non-transgenic plants were made. Both the ALS and GUS genes were shown to be transmitted to progeny by functional assays (see Table 4). For both cell lines, Chi-square analysis indicates that the GUS gene is being transmitted as a single functional locus. ALS data was only collected for cell line T21-1. For this line, ALS is also transmitted as a single functional locus, and is tightly linked to the GUS gene.

**TABLE 4**

| Female Parent | Male Parent | Cell Line | Tissue | #POS | #NEG | Chi-Square |
|---|---|---|---|---|---|---|
| Hi-II | R0237A | T21-1 | 8d endosperm | 39 | 33 | 0.35(1:1) |
| | | | 13d callus | 32 | 28 | 0.27 (1:1) |
| | | | Seedling Leaves | 5 | 5 | 0.10 (1:1) |
| R0237A | R0237A | T21-1 | Seedling Leaves | 27 | 10 | 0.01 (3:1) |
| Hi-II | R0252B | T17-1 | 8d endosperm | 43 | 38 | 0.28 (1:1) |
| | | | Seedling Leaves | 10 | 15 | 0.64 (1:1) |

For endosperm and seedling leaf data, "POS" means blue upon incubation in X-gluc which indicates expression of the GUS gene. For "13d callus," "POS" means callus formed within 13 days after explanting 8d old immature embryos onto N6 1-0-25-Ag-C20 medium containing 20ppb chlorsulfuron which indicates expression of the ALS gene.

After 13 days (13d callus) on 20 ppb chlorsulfuron media, 28 of the 32 positive calli and all 28 of the non-callusing embryos were tested for GUS expression with X-gluc. All of the chlorsulfuron resistant calli were GUS positive, and all of the chlorsulfuron sensitive embryos were GUS negative. This indicates tight linkage of the functional ALS and GUS genes in cell line T21-1.

Yates' correction factor for small sample sizes was used in the calculations.

Sixty-five days after bombardment, two additional independent chlorsulfuron-resistant cell lines were identified. Samples from each turned dark blue within hours after incubation in X-gluc, indicating stable transformation and expression of the GUS gene from pMON19433. No plants were regenerated from these cell lines.

### EXAMPLE 6

Immature embryos of the wheat cultivar Hartog were isolated from Hartog spikes and cultured on a modified MS medium. These immature embryos were cultured on the modified MS medium for four days after isolation. The embryos were then bombarded with pMON8678 DNA, as previously described, which were precipitated onto M10 tungsten particles and bombarded with the modified Biolistic® PDS 1000 particle gun.

Transient GUS expression was monitored by placing the embryon into X-gluc two days after bombardment, incubating at 37°C for about 24 hours, fixing in FAA, and then counting the number of blue spots per embryo. The embryos subjected to the four day culture pre-bombardment treatment averaged approximately twenty-four blue spots per embryo, whereas less than one blue spot per embryo was observed when freshly isolated immature embryos were similarly transformed. Therefore, it is apparent that the four day culturing period is useful to improve and enhance the transformation frequency of a variety of monocotyledonous plant species.

### BIBLIOGRAPHY

Abbe, E.C., Stein O.L. (1954) The origin of the shoot apex in maize:embryogeny. Am J. Bot. 41:285-293.
Armstrong C.L., Green C.E., and Phillips R.L. (1991) Development and availability of germplasm with high type-II culture formation response. Maize Genetics Cooperation Newsletter, 65:92-93.
Callis, J., Fromm M., and Walbot, V. 1987. Introns increase gene expression in cultured maize cells. Genes and Develop. 1:1183-1200.
Chu, C.C., Wang, C.C., Hsu, C., Yin K.C., Chu, C.Y., and Bin, F.Y., (1975) Establishment of an efficient medium for anther culture of rice through comparative experimentation on nitrogen sources, Scientia Sinica, Vol. 18:659-668.
Dekeyser, R.A., B. Claes, R.M.U. De Rycke, M.E. Habets, M.C. Van Montagu, and A.B. Caplan. 1990. Transient gene expression in intact and organized rice tissues. The Plant Cell 2:591-602.
Fraley, R.T., S.G. Rogers, R.B. Horsch, P.R. Sanders, J.S. Flick, S.O. Adams, M.L. Bittner, L.A. Brand, C.L. Fink, J.S. Fry, G.R. Galluppi, S.B. Goldberg, N.L. Hoffmann, and S.C. Woo. (1983). Expression of bacterial genes in plant cells. Proc. Natl. Acad. Sci. USA 80:4803-4807.
Fromm, M.E., F. Morrish, C. Armstrong, R. Williams, J. Thomas, and T.M. Klein. 1990. Inheritance and expression of chimeric genes in the progeny of transgenic maize plants. Bio/Technology 8:833-839.
Goff, S.G., Klein, T.M., Roth, B.A., Fromm, M.E., Cone, K.C., Radicella, J.P. and Chandler, V.L. (1990) Transactivation of anthocyanin biosynthetic genes following transfer of β-regulatory genes into maize tissues. EMBO J. 9:2517-2522.
Goff, S.G, Cone, K.C., and Fromm M.E. (1991) Identification of functional domains in the maize transcriptional activator C1 Comparison of wild-type and dominant inhibitor proteins. Genes and Development 5:298-309.
Gordon-Kamm, W.J., T.M. Spencer, M.L. Mangano, T.R. Adams, R.J. Danies, W.G. Start, J.V. O'Brien, S.A. Chambers, W.R. Adams, Jr., N.G. Willetts, T.B. Rice, C.J. Mackey, R.W. Krueger, A.P. Kausch, and P.G. Lemaux. 1990. Transformation of maize cells and regeneration of fertile transgenic plants. The Plant Cell 2:603-618.
Green, C.E. and Phillips, R.L. 1975. Plant regeneration from tissue cultures of maize. Crop Sci. 15:417-421.
Haughn, G.W., Smith J., Mazur, B. and Sommerville, C. 1987. Transformation with a mutant Arabidopsis acetolactate synthase gene renders tobacco resistant to sulfonylurea herbicides. Mol. Gen. Genet. 211:266-271.
Jefferson, R.A., T.A. Kavanaugh and M.W. Bevan. 1987. GUS fusions: β-glucuronidase as a sensitive and versatile gene fusion marker in higher plants. EMBO J. 6:3901-3907.
Kaeppler, H.F., G. Weining, D.A. Somers, H.W. Rines and A.F. Cockburn. 1990. Silicon carbide fiber-mediated DNA delivery into plant cells. Plant Cell Rep. 9:415-418.
Klein, T.M., M. Fromm, A. Weissinger, D. Tomes, S. Schaff, M. Sletten and J.C. Sanford. 1988. Transfer of foreign genes into intact maize cells with high-velocity microprojectiles. Proc. Natl. Acad. Sci. 85:4305-4309.
Klein, T.M., B.A. Roth, and M.E. Fromm. 1989. Regulation of anthocyanin biosynthetic genes introduced into intact maize tissues by microprojectiles. Proc. Natl. Acad. Sci. 86:6681-6685.
Lee, K.Y., Townsend, J., Tepperman, J., Black, M., Chui, C.F., Mazur, B., Dunsmir, P., and Bedbrook, J. 1988. The molecular basis of sulfonylurea resistance to tobacco. EMBO J. 7:1241-1248.
Lu, C.Y., Chandler, S.F., Vasil, I.K. 1984. Somatic embryogenesis and plant regeneration from cultured immature embryos of rye (Secale cereale L.). J. Plant Physiol. 115:237-244.
Maddock, S.E., Lancaster V.A., Risiott R., and Franklin J. 1983. Plant regeneration from cultured immature embryos and inflorescence of 25 cultivars of wheat (Triticum aestivum L.). J. Exp. Bot. 34:915-926.
McCabe, D.E., Swain, W.F., Martinell, B.J., and Christou, P. 1988. Stable transformation of soybean (Glycine max) by particle acceleration. Bio/Technology 6:923-926.
Neuffer, M.G. 1982. Growing maize for genetic purposes. In: Maize for Biological Research (W.F. Sheridan, Ed.). University Press, University of North Dakota, Grand Forks, North Dakota.
Oard, J.H., D.F. Paige, J.A. Simmonds, and T.M. Gradziel. 1990. Transient gene expression in maize, rice, and wheat cells using an air gun apparatus. Plant Physiol. 92:334-339.
Odell, J.T., F. Nagy and N.H. Chua (1985). Identification of DNA sequences required for activity of the cauliflower mosaic virus 35S promoter. Nature 313:810-812.
Peng, J. and Hodges, T.K. 1989. Genetic analysis of plant regeneration in rice (oryza sativa L.). In Vitro Cellular and Developmental Biology 25:91-94.
Potrykus, Ingo, 1990. Gene Transfer to Cereals: An Assessment. Bio/Technology 6:535-542.
Sambrook, J., Fritsch, E.F., and Maniatis T. Molecular cloning: A laboratory manual. (1989) Cold Spring Harbor Laboratory Press, Cold Spring Harbor, New York.
Thomas, M.R, Scott, K.J. 1985. Plant regeneration by somatic embryogenesis from callus initiated from immature embryos and immature inflorescence of Hordeum vulgare L. J. Plant Physiol. 121:159-169.
Topfer, R., B. Gronenborn, J. Schell, and H.H. Steinbiss. 1989. Uptake and transient expression of chimeric genes in seed-derived embryos. The Plant Cell 1:133-139.
Vieira, J. and J. Messing. 1982. The pUC plasmids, an M13mp7-derived system for insertion mutagenesis and sequencing with synthetic universal primers. Gene 19,259-268.

## Claims

1. A method for delivering foreign DNA into immature embryos, said method comprising:
isolating immature embryos from a fertilized corn ear;
culturing said immature embryos on a medium capable of inducing callus formation; and
introducing foreign DNA into said cultured immature embryos.

2. The method of claim 1 further comprising removing said cultured immature embryos from said culture medium prior to said introduction of said foreign DNA.

3. The method of claim 1 wherein said immature embryos are cultured from 1 to 14 days prior to said introduction of said foreign DNA.

4. The method of claim 1 wherein said immature embryos are cultured from 1 to 6 days prior to said introduction of said foreign DNA.

5. The method of claim 1 wherein said immature embryos are cultured from 1 to 4 days prior to said introduction of said foreign DNA.

6. The method of claim 1 wherein said culture medium for said culturing step is N6 1-100-25-Ag medium containing from about 10 µM to about 100 µM silver nitrate.

7. The method of claim 1 wherein said foreign DNA is introduced to said cultured immature embryos by either electroporation or microprojectile bombardment.

8. The method of claim 1 wherein said immature embryo is isolated from a corn genotype capable of efficient regeneration from immature embryos.

9. The method of claim 1 wherein said immature embryos are isolated from a fertilized corn ear from a high type-II genotype derived from inbred corn lines A188 and B73.

10. A method for preparing corn immature embryos for the introduction of foreign DNA comprising:
isolating immature embryos from a fertilized corn ear; and
culturing said immature embryos on a medium capable of inducing callus formation.

11. The method of claim 10 further comprising removing said cultured immature embryos from said culture medium prior to the introduction of foreign DNA.

12. The method of claim 10 wherein said immature embryos are cultured from 1 to 14 days prior to the introduction of foreign DNA.

13. The method of claim 10 wherein said immature embryos are cultured from 1 to 6 days prior to the introduction of foreign DNA.

14. The method of claim 10 wherein said immature embryos are cultured from 1 to 4 days prior to the introduction of foreign DNA.

15. The method of claim 10 wherein said culture medium for said culturing step is N6 1-100-25-Ag medium containing from about 10 µM to about 100 µM silver nitrate.

16. The method of claim 10 wherein said immature embryo is isolated from a corn genotype capable of efficient regeneration from immature embryos.

17. The method of claim 10 wherein said immature embryos are isolated from a fertilized corn ear from a high type-II genotype derived from inbred corn lines A188 and B73.

18. A method for introducing genes into monocotyledonous plants comprising:
isolating immature embryos from a fertilized reproductive organ of a monocotyledonous plant;
culturing said immature embryos on a culturing medium capable of inducing callus formation;
transforming said cultured immature embryos with a DNA sequence comprising a foreign gene under the transcriptional control of regulatory sequences capable of causing expression in plant cells;
culturing said transformed cultured immature embryos on a medium capable of inducing embryogenic callus formation; and
regenerating said embryogenic callus into whole monocotyledonous plants.

19. The method of claim 18 wherein said immature embryos are cultured from 1 to 14 days prior to said transformation step.

20. The method of claim 18 wherein said immature embryos are cultured from 1 to 6 days prior to said transformation step.

21. The method of claim 18 wherein said immature embryos are cultured from 1 to 4 days prior to said transformation step.

22. The method of claim 18 wherein said culture medium is N6 1-100-25-Ag medium containing from about 10 µM to about 100 µM silver nitrate.

23. The method of claim 18 wherein said method of transformation is selected from the group consisting of electroporation and microprojectile bombardment.

24. The method of claim 18 wherein said monocotyledonous plant is a species from the Gramineae family.

25. The method of claim 25 wherein said species is selected from the group consisting of corn, wheat, barley, rye, rice and sorghum.
